# EUROPEAN PATENT SPECIFICATION

(11) **EP 2 051 695 B1**
(45) Date of publication and mention of the grant of the patent: **04.09.2013**
(21) Application number: 07836050.0
(22) Date of filing: 10.07.2007
(51) Int. Cl.: A61K 9/06, A61K 9/00, A61K 9/12, A61F 2/00

(54) **IMPLANTABLE SUSTAINED DRUG DELIVERY DEVICE FOR RELEASING ACTIVE AGENTS TO THE OSTEOMEATAL COMPLEX**
IMPLANTIERBARE VORRICHTUNG ZUR VERZÖGERTEN FREISETZUNG VON WIRKSTOFFEN IN DEN OSTIOMEATALEN KOMPLEX
DISPOSITIFS A IMPLANTATION QUI MONTRENT UNE LIBERATION PROLONGEE DU PRINCIPE ACTIF AU COMPLEXE OSTIOMEATIQUE

(30) Priority: 10.07.2006 US 819825 P; 09.07.2007 US 775157
(43) Date of publication of application: 29.04.2009
(73) Proprietor: Intersect ENT, Inc., Palo Alto, CA 94303 (US)
(72) Inventor: ARENSDORF, Patrick, A., Palo Alto, CA 94303 (US); BIGGS, Danielle, L., Hoover, AL 35244 (US); BRENNEMAN, Rodney, San Juan Capistrano, CA 92675 (US); DOWNIE, David, B., "deceased" (US); EATON, Donald, J., Los Altos, CA 94022 (US); TICE, Thomas, R., Indian Springs, AL 35124 (US)
(74) Representative: Price, Nigel John King
(86) International application number: PCT/US2007/015813
(87) International publication number: WO 2008/008389

(56) References cited:
- WO-A-2004/082525
- WO-A-2006/107957
- US-A1- 2007 005 094
- US-A1- 2009 017 090

## Description

### FIELD

The devices, methods, and kits described here are in the field of medical devices and local drug delivery to treat sinusitis and its related respiratory conditions. Specifically, the treatment of osteomeatal complex inflammation is described.

### BACKGROUND

The osteomeatal complex (OMC) is a key area in the pathogenesis of sinusitis. The OMC includes the middle meatus and the narrow channels that provide the pathway for mucociliary clearance and ventilation of the anterior ethmoid, maxillary, and frontal sinuses. Thus, relatively minor swelling in this area, such as that associated with upper respiratory tract infections or allergic rhinitis, may lead to obstruction of any one or combination of these sinus cavities. As a result of blockage in the OMC, sinusitis may develop due to the accumulation of mucus, inflammatory cells, and bacteria, presence of low oxygen tension, and impaired immune responses in the OMC and surrounding tissues.

Medical treatment regimens used for sinusitis may be used to treat OMC inflammation. Typical medical treatment regimes may include a combination of oral antibiotics, topical or oral decongestants, topical steroid nasal sprays or solutions, or injectable oral steroids such as prednisone. Systemic methods (e.g., oral and injectables) commonly have significant disadvantages relating to side effects from the exposure of the entire body to the effects of the active agent. Topical methods (e.g., nasal sprays and other solutions) commonly have disadvantages relating to the limited site availability of the active agents both nasally and at the inflamed OMC anatomy (typically less than a 30 percent nasal drug delivered dose efficiency, and presumably far less at the specific OMC anatomy) as well as a short drug residence time at the inflamed site due to the effect of mucociliary clearance (typically less than 30 minutes dose residence time within the nasal passage).

When medical therapy fails, surgical treatment such as functional endoscopic sinus surgery (FESS) may be an alternative. The goal of FESS, and of sinus surgery generally, is to improve the drainage of the sinuses by enlarging the ostia of the maxillary and frontal sinuses, and opening the ethmoid sinus area by removing the ethmoid air cells under direct visualization. However, surgery itself creates inflammation, which can lead to postoperative fibrosis, stenosis, and/or polyposis that frequently obstructs the newly opened sinuses, requiring the surgeon to reoperate to revise the ostia and insert stenting devices to keep sinus ostia patent. Even in the resected post-surgical anatomy, access to many of the inflamed regions of the OMC remains difficult.

Other methods of post-surgical adjunctive drug delivery have required endoscopic placement of various drug hydrated packing materials, typically resident for a week or less at a time, and presumably delivering drug for shorter periods than the packing material residence time as only an acute one-time instillation of active agents is possible (as described in Shikani, AH, Use of Antibiotics for Expansion of the Merocel Packing Following Endoscopic Sinus Surgery, ENTJournal 75 (8): 524-527 (1996)). Such approaches are unlikely to allow consistent or controllable sustained release of active agents. Still other described approaches involve acute endoscopic injection of active agent releasing depots, as in the intramuscular injection of steroids (e.g., triamcinolone acetonide suspensions such as Kenalog) into adjacent soft tissue. These approaches require invasive rather than topical methods (e.g., intramuscular injection at a site), and neither treat the OMC region directly (which is primarily thin tissue membranes over bony cavities not suitable for an intramuscular depot) nor treat without significant collateral systemic and adjacent tissue exposure and adverse effects (e.g., the use of Kenalog in the paranasal and sinus anatomy has been linked to both systemic adrenal suppression as well as cases of ipsilateral blindness, in many cases permanent).

Consequently, new devices, methods, and kits to locally administer and provide sustained release of active agents directed to the OMC for treating sinusitis and its related respiratory conditions are desirable.

WO-A-2004/082525 discloses biodegradable implants for treating sinusitis. The biodegradable implants have a size, shape, density, viscosity, and/or mucoadhesiveness that prevents them from being substantially cleared by the mucociliary lining of the sinuses during the intended treatment period. The biodegradable implants include a sustained release therapeutic, e.g., an antibiotic, a steroidal anti-inflammatory agent, or both. The biodegradable implants may take various forms, such as rods, pellets, beads, strips, or microparticles, and may be delivered into a sinus in various pharmaceutically acceptable carriers.

WO-A-2006/107957 is, at most, admissible as prior art against the present application under Article 54(3) EPC. It discloses paranasal sinus devices for treating paranasal sinus conditions. The devices include a cavity member, and nasal portion. One or more of the cavity member, ostial member and nasal portion may deliver an active agent for sustained release to treat the paranasal sinus condition. Exemplary paranasal sinus conditions are sinus inflammation due to functional endoscopic sinus surgery (FESS) and rhinosinusitis.

### SUMMARY

According to the present invention there is provided an implantable sustained drug delivery device for treating sinusitis and related respiratory conditions, comprising:
a cannula with a pointed end for piercing and cannulating one or more structures of the osteomeatal complex and one or more fixation elements for securing the cannula within the one or more structures of the osteomeatal complex; and
wherein the cannula includes a therapeutically effective amount of an active agent and is constructed and arranged to deliver the active agent for local sustained release to the osteomeatal complex.

The devices, methods, and kits described here are generally used to treat patients with sinusitis and related respiratory conditions. The related respiratory condition typically treated is osteomeatal complex inflammation. As used herein, the phrases "osteomeatal complex inflammation" or "OMC inflammation" refer to any reaction of tissue within the osteomeatal complex and its constituent adjacent anatomy that involves the inflammatory response. The inflammation may be caused by processes such as allergy (hypersensitivity), bronchitis, asthma, injury to mucosa within the OMC due to, e.g., trauma; surgery; infection by bacteria, viruses, or fungi; chemicals or drugs; cystic fibrosis, and benign or malignant tumors.

The devices described here are generally formed in such a way to locally deliver one or more active agents to the OMC. The devices may be made from a biodegradable polymer, a nonbiodegradable polymer, a metal, or a combination thereof. The active agents that may be delivered include, but are not limited to, anticholinergic agents, antihistamines, anti-infective agents, anti-inflammatory agents, antiscarring or antiproliferative agents, chemotherapeutic or antineoplastic agents, cytokines, decongestants, healing promotion agents and vitamins, hyperosmolar agents, immunomodulator or immunosuppressive agents, leukotriene modifiers, mucolytics, narcotic analgesics, small molecules, tyrosine kinase inhibitors, peptides, proteins, nucleic acids, vasoconstrictors, and combinations thereof. In one variation, the active agent may be included in the biodegradable polymer or in a coating on the device. In another variation, the active agent may be encapsulated in microspheres or other micro-particles that are components of the device.

In addition to the drug delivery function described above, the devices may optionally perform one or more mechanical functions and may also have features configured to enhance their utility. Optional mechanical functions may include the stabilization of the natural or post-surgical anatomy, piercing and/or cannulation providing access conduits to, from and within the OMC anatomy and the sinuses, prevention of tissue adhesions as a physical adhesion barrier, coating and/or tissue separating spacer, and the replacement of natural anatomical features removed by surgery or disease processes. The devices described here have features configured to be actively fixed to one or more tissues of the OMC and may be configured to either maintain their position within the OMC or to maintain contact with at least some of the various parts of the OMC anatomy using these features or other material properties such as mucoadhesion. Other devices may have features adapted to fill the space or maintain a separation between various parts of the OMC anatomy, e.g., between the uncinate process and middle turbinate, between the ethmoid bulla and the middle turbinate or between the lateral nasal wall and the middle turbinate. The devices may optionally include a portion that extends and delivers an active agent into a sinus ostium, a sinus cavity, and/or the nasal passage. If included, the active agent delivered to the sinus ostium, sinus cavity, and/or the nasal passage may be the same or different from the active agent delivered to the osteomeatal complex. The optional portion itself that extends into the sinus ostium, sinus cavity, and/or nasal passage may also be configured to have a function other than drug delivery, e.g., stabilization or lateralization of the middle turbinate, cannulation of the ostium or sinus cavity, etc. The devices may also change their insertion configuration and deploy through expansion, deformation or self-assembly into their final intended configuration after their placement. Kits may also be formed by packaging a device with one or more delivery conduits, insertion devices, or deployment devices, or by packaging together with one or more of various types of devices including functions supporting or enabling the access, placement, adjustment, expansion or deployment, and removal of device(s).

The described devices may be useful in surgical, non-surgical, and other therapeutic interventions related to the OMC to restore anatomical function and treat sinusitis and related respiratory conditions. Accordingly, the devices may be used to support sinus and nasal surgery, reduce the need for surgical revision, and/or prevent, delay, or reduce recurrence of sinusitis and related respiratory conditions.

### BRIEF DESCRIPTION OF THE DRAWINGS

FIGS. 1A-1E show cross-sectional views of pre-surgical (FESS) sinus and OMC anatomy.

FIG. 2 is a flowchart showing mucociliary flow into and out of the OMC.

FIG. 3 illustrates a cross-sectional view of post-surgical (FESS) sinus and OMC anatomy.

FIGS. 4A-4E show multiple views of a micro-cannula for piercing and/or cannulating the OMC. FIG. 4A is a perspective view; FIG. 4B a frontal view; FIG. 4C an end view; FIG. 4D a side view; and FIG. 4E a side cross-sectional view.

FIGS. 5A-5E show multiple views of a micro-needle for piercing and/or cannulating the OMC. FIG. 5A is a perspective view; FIG. 5B is a frontal view; FIG. 5C is an end view; FIG. 5D is a side view; and FIG. 5E is a side cross-sectional view.

FIGS. 5F-5H are perspective views of the various tip configuration that may be used with the micro-needle of FIG. 5A.

FIGS. 6A - 6E show multiple views of a locking deformation device for piercing and/or cannulating the OMC. FIG. 6A is a perspective view; FIG. 6B is a frontal view; FIG. 6C is an end view; FIG. 6D is a side view; FIG. 6E is a side cross-sectional view; and FIG. 6F shows the locking deformation rod of FIG. 6A within the OMC anatomy.

FIGS. 7A-7H show perspective views of various active fixation elements.

FIGS. 7I-7L show cross-sectional views of various ridge configurations.

FIG. 8 depicts a cross-sectional view of a device having an optional portion extending within multiple ethmoid air cells.

FIGS. 9, 10 and 11A-11B show various flow through features that may included in the described devices. FIG. 9 shows a porous bead; FIG. 10 a cylindrical device with a plurality of end and side apertures; and FIGS. 11A - 11B an egg carton-like surface design.

### DETAILED DESCRIPTION

The devices, methods, and kits described herein relate to the delivery of active agent(s) to the OMC to treat OMC inflammation. The OMC includes the middle meatus and the area adjacent to and including the narrow channels that provide mucociliary clearance and ventilation of the anterior ethmoid, maxillary, and frontal sinuses. As used herein, the terms "osteomeatal complex" or "OMC" refer to the anatomical space bounded laterally by the nasal wall, the lateral surface of the ethmoid bulla and anterior ethmoid cells, medially by the lateral surface of the middle turbinate, superiorly by (and including) the frontal recess up to the frontal sinus ostia, inferiorly by the superior surface of the inferior turbinate, posteriorly by the termination of the infundibular groove and the junction of the anterior surface of the basal lamella with the anterior ethmoid cells, and anteriorly by the anterior side of the uncinate process (which is also included in its entirety). If the ethmoid cells and bulla have been resected, then the OMC is bounded laterally by the nasal wall and medially by the lateral surface of the remnant middle turbinate. If the middle turbinate has also been resected, then the OMC is bounded medially by the nasal septum. If the uncinate process has been resected, then the OMC is bounded anteriorly by the anterior edge of the middle turbinate.

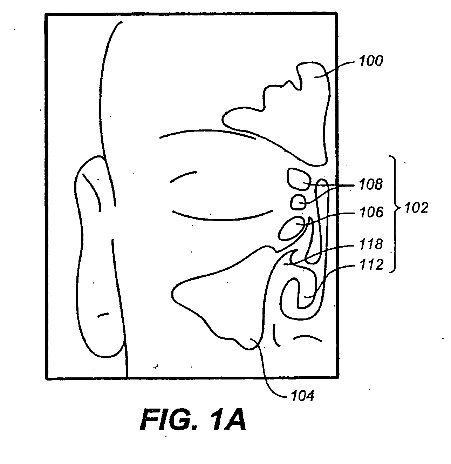

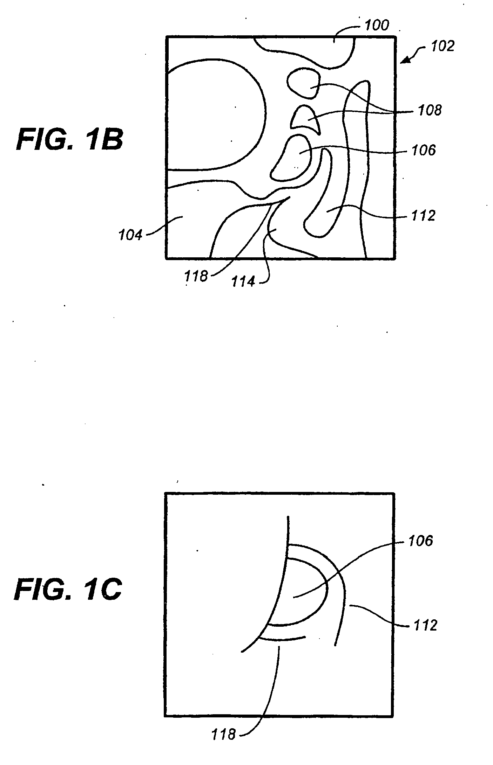

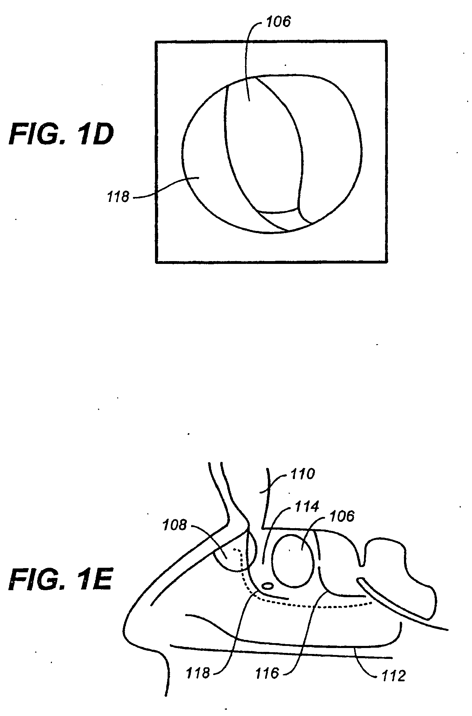

Shown in FIGS. 1A-1E are pre-surgical (FESS) views of the sinus anatomy. Specifically, FIG. 1A depicts a coronal sinus view of the pre-surgical sinus anatomy, FIG. 1B is an exploded coronal view of the OMC, FIG. 1C is a schematic view of the OMC, FIG. 1D is an endoscopic view of the OMC, and FIG. 1E is a sagittal view of the sinus OMC.

Specifically, shown in FIG. 1A, is frontal sinus (100), OMC (102), and the maxillary sinus (104). FIG. 1B is a partial exploded coronal view of the OMC (102). As noted above, pre-surgically (when neither the middle turbinate or uncinate process has been resected) the OMC refers to the anatomical space bounded laterally by the nasal wall and the lateral surface of the ethmoid bulla (106) and anterior ethmoid cells (108), medially by the lateral surface of the middle turbinate, superiorly by (and including), the frontal recess (110) up to the frontal sinus ostia, inferiorly by the superior surface of the inferior turbinate (112), posteriorly by the termination of the infundibular groove (114) and the junction of the anterior surface of the basal lamella (116) with the anterior ethmoid cells (108), and anteriorly by the anterior side of the uncinate process (118).

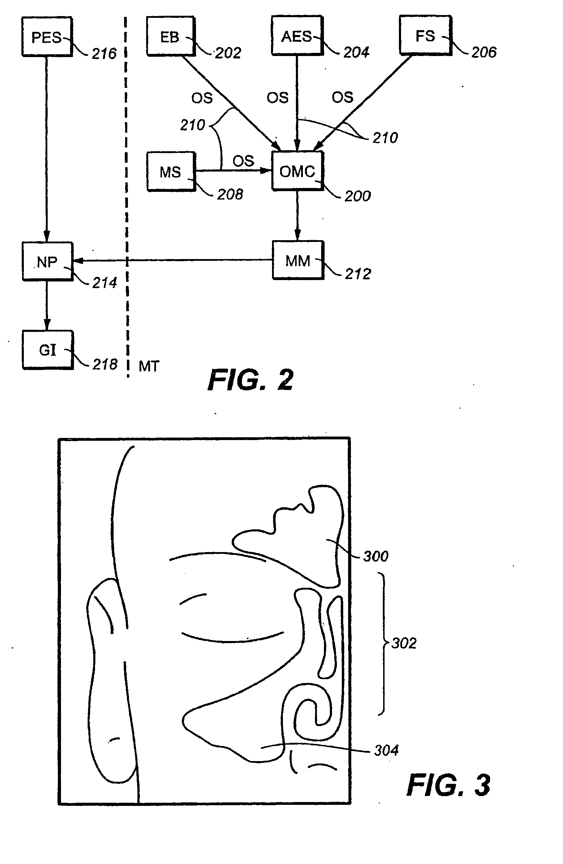

FIG. 2 is a flowchart illustrating the mucocilliary flow into and out of the OMC (200). Specifically, mucus flows from the ethmoid bulla (202), the anterior ethmoid cells (204), frontal sinus (206), and maxillary sinus (208) to enter the OMC through their respective ostia (210). Mucocilliary flow out of the OMC enters the middle meatus (212), which in turn enters the nasal passage (214). Flow from the posterior ethmoid cells (216) also enters the nasal passage (214). From the nasal passage, mucus then enters the gastrointestinal tract (218) via the nasopharynx, pharynx, hypopharynx, and esophagus.

FIG. 3 provides a coronal view of post-surgical (FESS) sinus anatomy. Shown there is frontal sinus (300), post-surgical OMC (302), and maxillary sinus (304). As noted above, ifthe middle turbinate has been resected, then the OMC is bounded medially by the nasal septum. If the uncinate process has been resected, then the OMC is bounded anteriorly by the anterior edge of the middle turbinate.

### I. DEVICES

Described here are devices for treating sinusitis and its related respiratory conditions. In general, the devices comprise a therapeutically effective amount of an active agent, which is delivered for local sustained release to the OMC (including its adjacent anatomy). The related respiratory condition may be selected from the group consisting, without limitation of the foregoing, of inflammation of the OMC, OMC or sinus cavity inflammation due to surgery, including functional endoscopic sinus surgery (FESS); respiratory infections; sinusitis (acute or chronic); rhinitis; allergic rhinitis; rhinosinusitis (acute or chronic); upper respiratory tract infections; otitis media; bronchitis; bronchiolitis; asthma; tonsillitis and other chronic diseases of the tonsils and adenoids; laryngitis; tracheitis; nasal and sinus polyposis; neoplasms of the large and small airways; and nasal, sinus, and nasopharynx tumors.

The devices may be of any dimension, and as further described below, may be formed to be solid, semi-solid, biodegradable, nonbiodegradable, mucoadhesive, expansive, porous or flow-through, etc.

The devices serve to pierce or cannulate, e.g. provide access and potentially conduits to, from, and within the OMC anatomy and the sinuses. The devices may optionally further serve mechanical functions such as stabilising
preventing tissue adhesions (e.g., by acting as a physical barrier or coating or as a tissue separating spacer), and replacing or substituting for natural anatomical features removed by surgery or disease processes. The devices may have optional portions that extend and deliver an active agent to the sinus ostium, a sinus cavity, and/or the nasal passage, which portion(s) may have a function other than drug delivery (e.g., including preventing or reducing stenosis of a sinus ostium by mechanical mechanisms). The devices may have features which provide for their fixation to any part of the OMC anatomy, for their deployment, expansion or deformation, and self-assembly into an intended configuration, as well as the locking or stabilization of that configuration, as ¨further described in the below examples. In addition to those devices and features described below, the devices may comprise those devices (or portions thereof) described in Applicants' copending U.S. Pat. App. Serial No. 11/398,342, filed on April 4, 2006 and entitled "Device and Methods for Treating Paranasal Sinus Conditions,". In these variations, the devices would be sized and shaped to fit within the OMC anatomy as described below.

### Active and Passive Fixation

The devices are configured for active fixation, which may be useful in positioning, deploying, and anchoring the devices to any part of the OMC anatomy. As used herein, the phrases "active fixation," "actively fixing," and the like refer to devices and methods that visually alter the OMC anatomy in some fashion (e.g., puncturing, piercing, clamping, stimulating tissue ingrowth, and the like). The phrases "passive fixation," or "passively fixing" and the like refer to devices and methods that do not visually alter the OMC anatomy (e.g., space filling, space fitting, friction fitting, tension fitting, mucoadhesion, etc.). Of course, a device configured for passive fixation may become an active fixation device if it begins to visibly alter the OMC anatomy. It should also be understood that devices may be configured for both active and passive fixation. For example, a device may comprise one portion designed to pierce an area of the OMC anatomy (active portion) and another portion designed to expand to fill some portion of the OMC anatomy (passive portion).

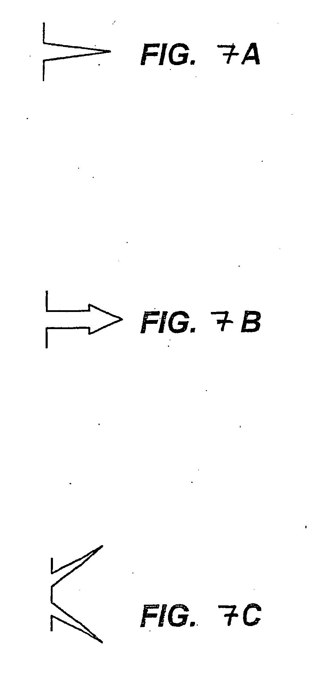

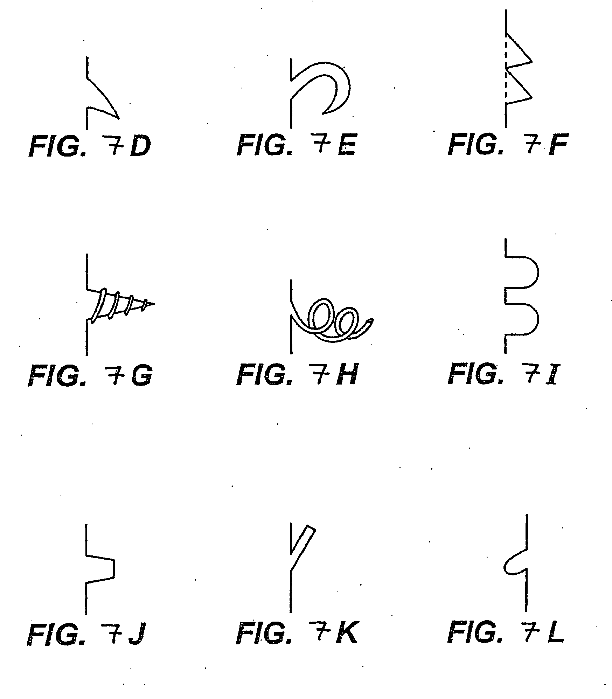

The devices include one or more elements that help to actively fix it to the sinus mucosa. For example, as shown in FIGS. 7A-7L, the active fixation elements may be one or more spies (7A), arrows (7B), opposed spikes (7C), barbs (7D), hooks (7E), triangular ridges (7F), screws (7G), springs (7H), and the like. In addition to being triangular in geometry, the ridges may also be formed to be round (7I), square (7J), directionally orientated or deployed (7K which can be inserted as a flat ridge and deploys directionally upon pulling the device backwards or proximally against the direction of insertion), concave (as in 16L, but also including other variations of the previous designs where concave versions of the aforementioned convex and protruding shapes might provide active fixation attributes through encouraging ingrowth, or alternatively, through passive fixation mechanisms), and the like. Furthermore, combinations of any number or all of the aforementioned active and passive fixation elements may also be used in the same devices. As previously noted, the devices may be of any dimension, and the above fixation techniques extend to microstructured and microparticle devices such as that formed by extrusion, molding, casting, and lithography techniques.

### Piercing and Cannulation

Devices that can pierce and/or cannulate the anatomy of the OMC are also described here. These devices may be configured for placement on or within the uncinate process, on or within the ethmoid bulla, on or within the anterior ethmoid cells, or on or within the middle turbinate.

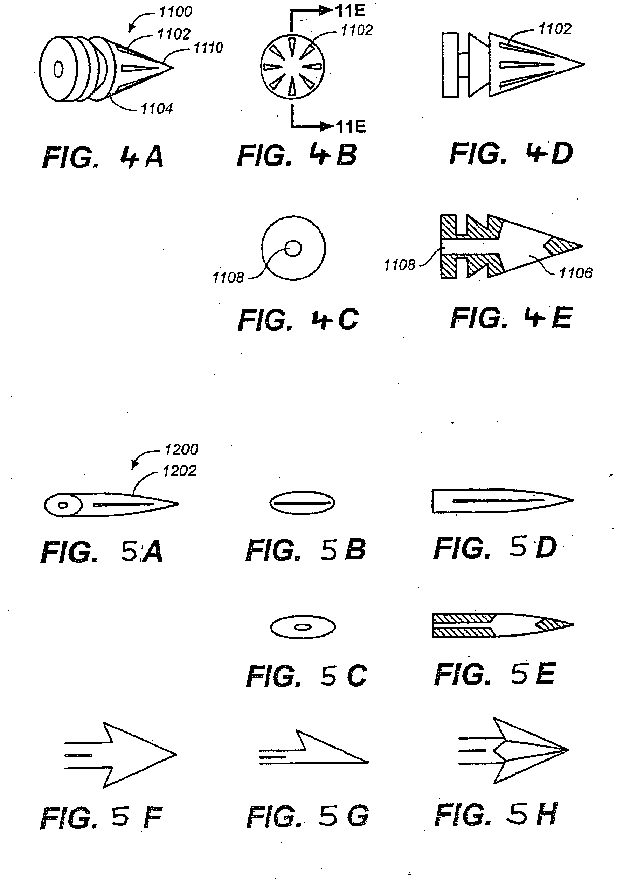

FIG. 4A is a perspective view of a micro-cannula (1100) configured for piercing a portion of the OMC anatomy. FIG. 4B shows a frontal view of the micro-cannula (1100) of FIG. 4A, while FIG. 4C shows an end view of the micro-cannula (1100). FIGS. 4D and 4E show side and cross-sectional side views of micro-cannula (1100) respectively. As shown in these figures, micro-cannula (1100) may include a plurality of openings (1102) within its distal portion (1104) that connect with space (1106) and lumen (1108). Although space (1106) is not shown as communicating with the tip (1110) of the micro-cannula, it can be designed as such if desired.

FIG. 5A is a perspective view of a micro-needle (1200). FIG. 5B is a front view of micro-needle (1200) and FIG. 5C is an end view. Similarly, FIGS. 5D and 5E are side and cross-sectional side views of the micro-needle respectively. The micro-needle can be of any gauge. To be in accordance with the present invention the micro-needle, for example the distal tip (1202) of the micro-needle may, is provided with one or more fixation elements as deemed necessary. For example, distal tip (1202) may configured as an arrow (FIG. 5F), barb (FIG. 5G), star (FIG. 5H), and the like.

While certain devices have been described, it should be understood that they are merely illustrative variations. Indeed, any device configured to pierce or cannulate a portion of the OMC anatomy may be suitable for use with the methods described herein. For example, the portion of the device configured to pierce (e.g., the device tip) may have a variety of configurations, e.g., the portion can have one piercing edge or two piercing edges. Furthermore, the devices are formed to locally or systemically release active agents themselves (e.g., be constructed of drug releasing materials, or including drug releasing components or coatings, such as biodegradable polymers) over a period of time.

### Locking or Deformation

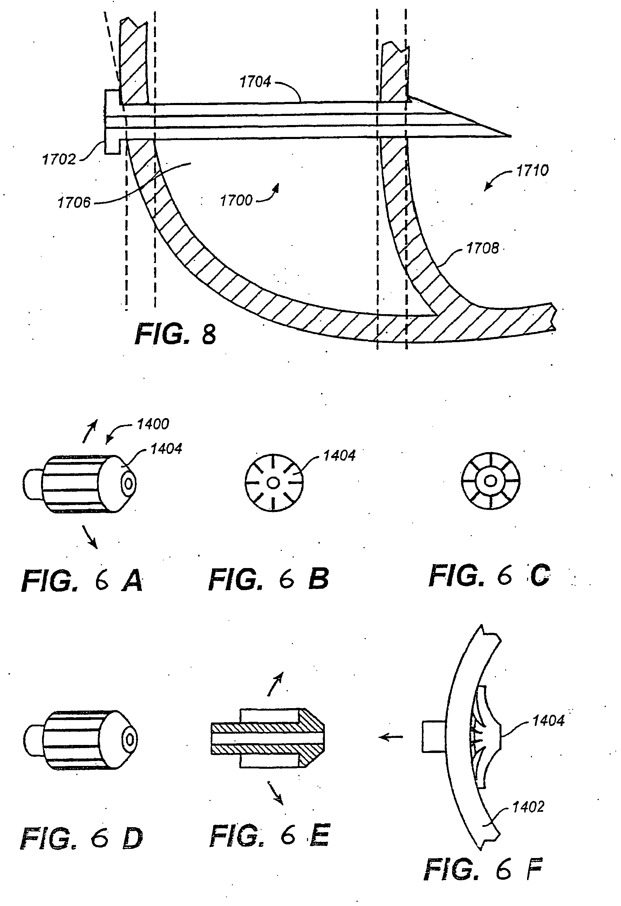

The devices may utilize looking or deformation features in order to stabilize or fix a device in a desirable configuration or anatomical position or to provide another mechanical function such as cannulation. One piercing and cannulation device variation with a locking feature is shown in FIGS. 6A-6F. Shown in FIG. 14A is a locking-deformation rod (1400). FIGS. 6B and 6C are frontal and end views of locking deformation rod (1400) respectively. FIGS. 6D and 6E are side and cross-sectional side views respectively. FIG. 6F shows an illustrative method of inserting the device in the OMC anatomy. As shown there in FIG. 6F the deformation rod (1400) is moved distally into and through the OMC anatomy (1402), and then pulled proximally back so that distal tip (1404) of deformation rod (1400) deforms, thereby locking the device in place.

Several alternative mechanical locking mechanisms may be used. Examples comprise tightening screw sleeves, opposing directional ridges, concentric tubes and interior friction (including where tapering or ridged tubes are used), rotating asymmetric interior members creating frictional opposition when locked (as with rotating oval cyclinders within the lumen of a surrounding round or oval member), protuberances extending from the interior or through the exterior of a device (as through portal windows) when locked, or individual member deformation (e.g., through tension, expansion or with compression), and the like. Such locking mechanisms may reversibly or irreversibly hold the device in the intended configuration or position.

### Device Extension Outside of the OMC

The devices may be designed to optionally include a portion or member that resides within a sinus ostia, a sinus cavity, the nasal passageway, and/or other areas beyond boundaries of the OMC. For example, as shown in FIG. 8, device (1700) may have an OMC portion (1702) and a sinus portion (1704) that extends into one or more sinus cavities. In FIG. 8, device (1700) pierces and cannulates the ethmoid bulla (1706) and septum (1708) between the ethmoid bulla (1706) and anterior ethmoid cell (1710) to create accessory openings for mucus drainage.

### Porosity and Flow- Through

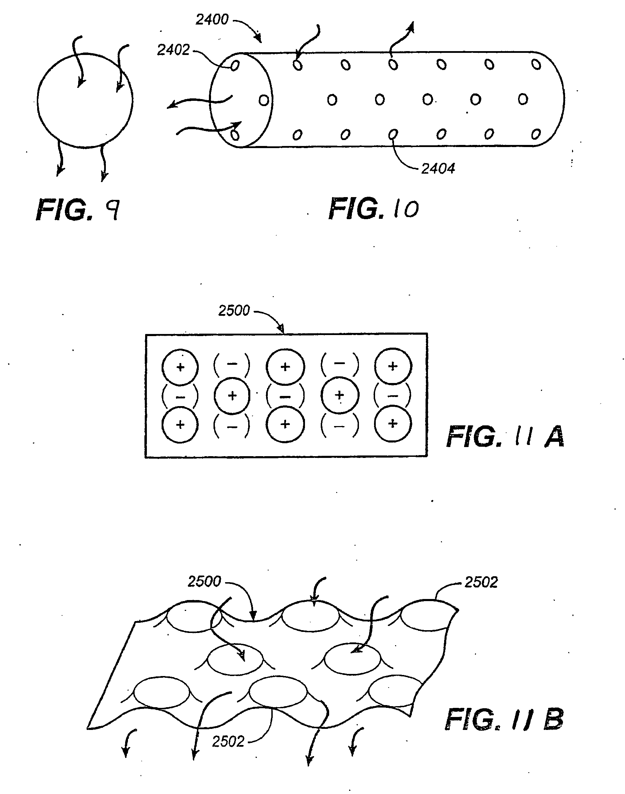

The devices may be configured to include various features that allow continued mucociliary clearance or features that promote flow of mucus through and/or around them. For example, as shown in FIG. 9, the device may comprise one or more porous beads (2300) that permit mucus flow therethrough, as shown by the arrows. The device may also be formed as perforated structures (e.g., holes, slots, etc.). Shown in FIG. 10 is a cylindrical device (2400) having a plurality of end apertures (2402) and side apertures (2404) interconnected by channels (not shown). As shown by the direction of the arrows, mucus may flow into a side aperture (2402) and out an end aperture (2402) and vice versa. In another variation, as shown in FIGS. 11A and 11B, the surface of the device may be textured to comprise a series of concavities 2500 (indicated by (-) in FIG. 11A) and convexities 2502 (indicated by (+) in FIG. 11A) such that mucus flows through the depressions formed by the concavities, as shown by the arrows in FIG. 11B. Other suitable structures for flow-through and continued mucociliary clearance include, but are not limited to, gels, sponges, porous monoliths, woven and nonwoven meshes, and the like. Illustrative, but non-limiting examples of suitable porosity and flowthrough materials for use with the methods and devices provided here are described generally, or conceptually, in the following references: Sarkar et al. Development and Characterization of a Porous Micro-patterned Scaffold for Vascular Tissue Engineering Applications. Biomaterials 27: 4775-4782 (2006); Rezwan et al. Biodegradable and Bioactive Porous Polymer/inorganic Composite Scaffolds for Bone Tissue Engineering. Biomaterials 27: 3413-3431 (2006); Svec F, Porous Monoliths. Recent Developments in LC Column Technology June: 2-6 (2003); Landgraf et al. Polymer Microcarrier Exhibiting Zero-Order Release. Drug Delivery Technology 3(1): 1-12 (2003); Lu et al. In vitro and in vivo degradation of Porous Poly(DL-lactic-co-glycolic acid) Foams. Biomaterials 21:1837-1845 (2000); Mooney et al. Novel Approach to Fabricate Porous Sponges of Poly(D,L-lactice-co-gylcolic acid) Without the use of Organic Solvents. Blomaterials 17:1417-1422 (1996); and Benson JR. Highly Porous Polymers. American Laboratory April (2003).

### Active Agents

Any active agent may be included in the devices described herein so long as they are suitable to treat OMC inflammation or a related respiratory condition and are capable of achieving the desired release kinetics. Inflammation is used generically herein to describe the complex biological response of vascular and surrounding tissues to harmful stimuli, such as pathogens, damaged cells, pro-inflammatory disorders, or irritants, and includes both a protective and healing function. Treatment of inflammation as described herein may include the treatment of either the harmful stimuli (such as with anti-microbial active agents), treatment of the cellular (such as active agents acting on inflammatory cell recruitment and infiltration (e.g., neutrophils, eisinophils, amongst others) of tissue from the vasculature to the mucosal wall) and molecular inflammatory responses (such as active agents impacting cell receptor, signal transduction, nuclear factor signaling, nucleic acid transcription or transrepression, translation, post-translational modification, exudative cytokine release and extracellular signaling by affected and relevant cells), and treatment and aid of the healing response (such as active agents either through supporting cellular regeneration of the original cell type (e.g., active agents for angeiogenesis and/or growth factors) or replacement of the injured tissue with scar tissue), as well as all acute, chronic, or traumatic manifestations thereof. Other active agents may be used to treat conditions or complications secondary to surgery, implantation of the devices, or other treatment, such as in post-surgical inflammation, inflammation due to foreign body reactions to the devices, and secondary infections or biofilm formation and microbial colonization of the devices themselves. In one variation, the active agent may be included in a coating on the device. In another variation, the active agent may be encapsulated in a microparticle (e.g., a hydrocolloid microparticle or a polymeric microparticle). The active agents that may be used in such a device include, but are not limited to, anticholinergic agents, antihistamines, anti-infective agents, anti-inflammatory agents, antiscarring or antiproliferative agents, chemotherapeutic/antineoplastic agents, cytokines such as interfereon and interleukins, decongestants, healing promotion agents and vitamins (e.g., retinoic acid, vitamin A, dexapanthenol, vitamin B, and their derivatives), hyperosmolar agents, immunomodulator/immunosuppressive agents, leukotriene modifiers, mucolytics, narcotic analgesics, small molecules, tyrosine kinase inhibitors, peptides, proteins, nucleic acids, vasoconstrictors, or combinations thereof. Anti-sense nucleic acid oligomers or other direct transactivation and/or transrepression modifiers of mRNA expression, transcription, and protein production may also be used. Anti-infective agents generally include antibacterial agents, antifungal agents, antiparasitic agents, antiviral agents, and antiseptics. Anti-inflammatory agents generally include steroidal and nonsteroidal anti-inflammatory agents.

Examples of antibacterial agents that may be suitable for use with the described methods and devices include, but are not limited to, aminoglycosides, amphenicols, ansamycins, β-lactams, lincosamides, macrolides, nitrofurans, quinolones, sulfonamides, sulfones, tetracyclines, vancomycin, and any of their derivatives, or combinations thereof. In one variation, β-lactams are the preferred antibacterial agents.

β-lactams that may be suitable for use with the described methods and devices include, but are not limited to, carbacephems, carbapenems, cephalosporins, cephamycins, monobactams, oxacephems, penicillins, and any of their derivatives. In one variation, penicillins (and their corresponding salts) are the preferred β-lactams.

The penicillins that may be suitable for use with the described methods and devices include, but are not limited to, amdinocillin, amdinocillin pivoxil, amoxicillin, ampicillin, apalcillin, aspoxicillin, azidocillin, azlocillin, bacampicillin, benzylpenicillinic acid, benzylpenicillin sodium, carbenicillin, carindacillin, clometocillin, cloxacillin, cyclacillin, dicloxacillin; epicillin, fenbenicillin, floxacillin, hetacillin, lenampicillin, metampicillin, methicillin sodium, mezlocillin, nafcillin sodium, oxacillin, penamecillin, penethamate hydriodide, penicillin G benethamine, penicillin G benzathine, penicillin G benzhydrylamine, penicillin G calcium, penicillin G hydrabamine, penicillin G potassium, penicillin G procaine, penicillin N, penicillin O, penicillin V, penicillin V benzathine, penicillin V hydrabamine, penimepicycline, phenethicillin potassium, piperacillin, pivampicillin, propicillin, quinacillin, sulbenicillin, sultamicillin, talampicillin, temocillin, and ticarcillin. In one variation, amoxicillin may be included in the paranasal sinus device. In another variation, the device includes ampicillin. Penicillins combined with clavulanic acid such as Augmenting (amoxicillin and clavulanic acid) may also be used.

Examples of antifungal agents suitable for use with the described methods and devices include, but are not limited to, allylamines, imidazoles, polyenes, thiocarbamates, triazoles, and any of their derivatives. In one variation, imidazoles are the preferred antifungal agents. Antiparasitic agents that may be employed include such agents as atovaquone, clindamycin, dapsone, iodoquinol, metronidazole, pentamidine, primaquine, pyrimethamine, sulfadiazine, trimethoprim/sulfamethoxazole, trimetrexate, and combinations thereof.

Examples of antiviral agents suitable for use with the described methods and devices include, but are not limited to, acyclovir, famciclovir, valacyclovir, edoxudine, ganciclovir, foscamet, cidovir (vistide), vitrasert, formivirsen, HPMPA (9-(3-hydroxy-2-phosphonomethoxypropyl)adenine), PMEA (9-(2-phosphonomethoxyethyl)adenine), HPMPG (9-(3-Hydroxy-2-(Phosphonomet- -hoxy)propyl)guanine), PMEG (9-[2-(phosphonomethoxy)ethyl]guanine), HPMPC (1-(2-phosphonomethoxy-3-hydroxypropyl)-cytosine), ribavirin, EICAR (5-ethynyl-1-beta-D-ribofuranosylimidazole-4-carboxamine), pyrazofurin (3-[beta-D-ribofuranosyl]-4-hydroxypyrazole-5-carboxamine), 3-Deazaguanine, GR-92938X (1-beta-D-ribofuranosylpyrazole-3,4-dicarboxami- -de), LY253963 (1,3,4-thiadiazol-2-yl-cyanamide), RD3-0028 (1,4-dihydro-2,3-Benzodithiin), CL387626 (4,4'-bis[4,6-d] [3-aminophenyl-N- -,N-bis(2-carbamoylethyl)-sulfonilimino]-1,3,5-triazin-2-ylamino-biphenyl- 2-,2'-disulfonic acid disodium salt), BABIM (Bis(5-Amidino-2-benzimidazoly-1]-methane), NIH351, and combinations thereof.

Examples of antiseptic agents suitable for use with the described methods and devices include, but are not limited to, alcohol, chlorhexidrine, iodine, triclosan, hexachlorophene, and silver-based agents( e.g., silver chloride, silver oxide, silver nanoparticles).

Typically, if inclusion of an anti-inflammatory agent is desired, a steroidal anti-inflammatory agent, e.g., a corticosteroid, is employed. Exemplary steroidal anti-inflammatory agents include 21-acetoxypregnenolone, alclometasone, algestone, amcinonide, beclomethasone, betamethasone, budesonide, chloroprednisone, clobetasol, clobetasone, clocortolone, cloprednol, corticosterone, cortisone, cortivazol, deflazacort, desonide, desoximetasone, dexamethasone, diflorasone, diflucortolone, difluprednate, enoxolone, fluazacort, flucloronide, flumethasone, flunisolide, fluocinolone acetonide, fluocinonide, fluocortin butyl, fluocortolone, fluorometholone, fluperolone acetate, fluprednidene acetate, fluprednisolone, flurandrenolide, fluticasone propionate, formocortal, halcinonide, halobetasol propionate, halometasone, halopredone acetate, hydrocortamate, hydrocortisone, loteprednol etabonate, mazipredone, medrysone, meprednisone, methylprednisolone, mometasone furoate, paramethasone, prednicarbate, prednisolone, prednisolone 25-diethylamino-acetate, prednisolone sodium phosphate, prednisone, prednival, prednylidene, rimexolone, tixocortol, triamcinolone, triamcinolone acetonide, triamcinolone benetonide, triamcinolone hexacetonide, any of their derivatives, and combinations thereof. In one variation, budesonide is included in the device as the steroidal anti-inflammatory agent. In another variation, the steroidal anti-inflammatory agent may be mometasone furoate. In yet another variation, the steroidal anti-inflammatory agent may be beclomethasone. In yet a further variation, the steroidal anti-inflammatory agent may be fluticasone propionate.

If a nonsteroidal anti-inflammatory agent is used, suitable agents include, but are not limited to, COX inhibitors (COX-1 or COX nonspecific inhibitors) (e.g., salicylic acid derivatives, aspirin, sodium salicylate, choline magnesium trisalicylate, salsalate, diflunisal, sulfasalazine and olsalazine; para-aminophenol derivatives such as acetaminophen; indole and indene acetic acids such as indomethacin and sulindac; heteroaryl acetic acids such as tolmetin, dicofenac and ketorolac; arylpropionic acids such as ibuprofen, naproxen, flurbiprofen, ketoprofen, fenoprofen and oxaprozin; anthranilic acids (fenamates) such as mefenamic acid and meloxicam; enolic acids such as the oxicams (piroxicam, meloxicam) and alkanones such as nabumetone) and selective COX-2 inhibitors (e.g., diaryl-substituted furanones such as rofecoxib; diaryl-substituted pyrazoles such as celecoxib; indole acetic acids such as etodolac and sulfonanilides such as nimesulide).

The chemotherapeutic/antineoplastic agents that may be used in the devices described here include, but are not limited to antitumor agents (e.g., cancer chemotherapeutic agents, biological response modifiers, vascularization inhibitors, hormone receptor blockers, cryotherapeutic agents or other agents that destroy or inhibit neoplasia or tumorigenesis) such as alkylating agents or other agents which directly kill cancer cells by attacking their DNA (e.g., cyclophosphamide, isophosphamide), nitrosoureas or other agents which kill cancer cells by inhibiting changes necessary for cellular DNA repair (e.g., carmustine (BCNU) and lomustine (CCNU)), antimetabolites and other agents that block cancer cell growth by interfering with certain cell functions, usually DNA synthesis (e.g., 6-mercaptopurine and 5-fluorouracil (5FU), antitumor antibiotics and other compounds that act by binding or intercalating DNA and preventing RNA synthesis (e.g., doxorubicin, daunorubicin, epirubicin, idarubicin, mitomycin-C and bleomycin) plant (vinca) alkaloids and other anti-tumor agents derived from plants (e.g., vincristine and vinblastine), steroid hormones, hormone inhibitors, hormone receptor antagonists and other agents which affect the growth of hormone-responsive cancers (e.g., tamoxifen, herceptin, aromatase ingibitors such as aminoglutethamide and formestane, trriazole inhibitors such as letrozole and anastrazole, steroidal inhibitors such as exemestane), antiangiogenic proteins, small molecules, gene therapies and/or other agents that inhibit angiogenesis or vascularization of tumors (e.g., meth-1, meth-2, thalidomide), bevacizumab (Avastin), squalamine, endostatin, angiostatin, Angiozyme, AE-941 (Neovastat), CC-5013 (Revimid), medi-522 (Vitaxin), 2-methoxyestradiol (2ME2, Panzem), carboxyamidotriazole (CAI), combretastatin A4 pzodrug (CA4P), SU6668, SU11248, BMS-275291, COL-3, EMD 121974, IMC-1C11, IM862, TNP-470, celecoxib (Celebrex), rofecoxib (Vioxx), interferon alpha, interleukin-12 (IL-12) or any of the compounds identified in Science Vol. 289, Pages 1197-1201 (Aug. 17, 2000), which is expressly incorporated herein by reference, biological response modifiers (e.g., interferon, bacillus calmette-guerin (BCG), monoclonal antibodies, interleukin 2, granulocyte colony stimulating factor (GCSF), etc.), PGDF receptor antagonists, herceptin, asparaginase, busulphan, carboplatin, cisplatin, carmustine, cchlorambucil, cytarabine, dacarbazine, etoposide, flucarbazine, flurouracil, gemcitabine, hydroxyurea, ifosphamide, irinotecan, lomustine, melphalan, mercaptopurine, methotrexate, thioguanine, thiotepa, tomudex, topotecan, trebsulfan, vinblastine, vincristine, mitoazitrone, oxaliplatin, procarbazine, streptocin, taxol or paclitaxel, taxotere, analogs/congeners, derivatives of such compounds, and combinations thereof.

Exemplary decongestants that may be incorporated in the OMC devices, include, but are not limited to, epinephrine, pseudoephedrine, oxymetazoline, phenylephrine, tetrahydrozolidine, and xylometazoline. Mucolytics that may be used include, but are not limted to, acetylcysteine, dornase alpha, and guaifenesin. Anti-histamines such as azelastine, diphenhydramine, and loratidine may also be used.

In those instances where it is desirable to remove water from tissue, e.g., to remove fluid from polyps or edematous tissue, a hyperosmolar agent may be employed. Suitable hyperosmolar agents include, but are not limited to, furosemide, sodium chloride gel, or other salt preparations that draw water from tissue or substances that directly or indirectly change the osmolarity of the mucous layer.

### Materials

When the devices are made with polymers, selection of the biodegradable or nonbiodegradable polymer to be employed will vary depending on the residence time and release kinetics desired, method of device delivery, particular therapeutic agent used, and the like. In all instances, the biodegradable polymer when degraded results in physiologically acceptable degradation products.

Suitable biodegradable and biocompatible polymers for use in making the OMC devices include, but are not limited to, polymers such as a poly(lactide); a poly(glycolide); a poly(lactide-co-glycolide); a poly(lactic acid); a poly(glycolic acid); a poly(lactic acid-co-glycolic acid); poly(lactide)/poly(ethylene glycol) copolymers; a poly(glycolide) /poly(ethylene glycol) copolymers; a poly(lactide-co-glycolide) /poly(ethylene glycol) copolymers; a poly(lactic acid) /poly(ethylene glycol) copolymers; a poly(glycolic acid) /poly(ethylene glycol) copolymers; a poly(lactic acid-co-glycolic acid) /poly(ethylene glycol) copolymers; a poly(caprolactone); poly(caprolactone) /poly(ethylene glycol) copolymers a poly(orthoester); a poly(phosphazene); a poly(hydroxybutyrate) or a copolymer including a poly(hydroxybutyrate); a poly(lactide-co-caprolactone); a polycarbonate; a polyesteramide; a polyanhidride; a poly(dioxanone); a poly(alkylene alkylate); a copolymer of polyethylene glycol and a polyorthoester; a biodegradable polyurethane; a poly(amino acid); a polyetherester; a polyacetal; a polycyanoacrylate; a poly(oxyethylene)/poly(oxypropylene) copolymer, or a blend or copolymer thereof. Biodegradable shape memory polymers, such as those commercialized by nmemoscience in Aachen, Germany, or those described in U.S. 5,189,110 or U.S. 5,139,832, may also be employed, as may shape memory configurations of the aforementioned co-polymers using multiple layers or adjacent coatings of differing co-polymers (as described in Venkatraman SS et al. Biodegradable Stents with Elastic Memory. Biomaterials 27: 1573-1578 (2006)). *See also,* Zheng et al. Shape Memory Properties of poly(D,L-lactide)/hydroxyapatite composites. Biomaterials 27: 4288-4295 (2006).

As used herein, a poly(lactide); a poly(glycolide); a poly(lactide-co-glycolide); a poly(lactic acid); a poly(glycolic acid); a poly(lactic acid-co-glycolic acid) will all be referred to as PLG, PLG polymers, or lactide/glycolide polymers. Lactide/glycolide polymers for the drug delivery devices and compositions of this invention are typically made by melt polymerization through the ring opening of lactide and glycolide monomers. Some polymers are available with or without carboxylic acid end groups. When the end group of the poly(lactide-co-glycolide), poly(lactide), or poly(glycolide) is not a carboxylic acid, for example, an ester, then the resultant polymer is referred to herein as blocked or capped. The unblocked polymer, conversely, has a terminal carboxylic group. In one variation, linear lactide/glycolide polymers are used; however, star polymers may be used as well. In other variations, high molecular weight polymers may be used to form the devices of this invention, for example, to meet strength requirements and extend bioabsorption time. In other instances, low molecular weight polymers may be used when resorption time and not material strength is important. The lactide portion of the polymer has an asymmetric carbon. Racemic DL-, L-, and D-polymers are commercially available to include in the devices of this invention. The L-polymers are more crystalline and resorb slower than DL- polymers. In addition to copolymers comprising glycolide and DL-lactide or L-lactide, copolymers of L-lactide and DL-lactide are also commercially available. Additionally, homopolymers of lactide or glycolide are commercially available. Star polymers of lactide or glycolide or lactide/glycolide copolymers are also commercially available.

In the case when the biodegradable polymer is poly(lactide-co-glycolide), poly(lactide), or poly(glycolide), the amount of lactide and/or glycolide in the polymer may vary. In one variation, the biodegradable polymer contains from about 0 to about 100 mole %, from about 40 to about 100 mole %, from about 50 to about 100 mole %, from about 60 to about 100 mole %, from about 70 to about 100 mole %, or from about 80 to about 100 mole % lactide, and from about 0 to about 100 mole %, from about 0 to about 60 mole %, from about 10 to about 40 mole %, from about 20 to about 40 mole %, or from about 30 to about 40 mole % glycolide, wherein the amount of lactide and glycolide is 100 mole %. In other variations, the biodegradable polymer may be poly(lactide), about 85:15 poly(lactide-co-glycolide), about 75:25 poly(lactide-co-glycolide), about 65:35 poly(lactide-co-glycolide), or about 50:50 poly(lactide-co-glycolide), where the ratios are mole ratios. When the biodegradable polymers are fibers, they may be made via an extrusion process. For example, the polymer may be extruded via a melt phase process to form a fiber with a suitable diameter. The fiber can than be further drawn down to smaller diameters, if desirable. The extrusion temperature will typically be above the melt temperature of the polymer, and will vary depending on the type of polymer chosen. The drawing process will typically involve drawing the polymer at a temperature above the glass transition temperature and then heat setting the polymer at a temperature between the glass transition temperature and the melting temperature. The fiber may be any suitable length. Fiber meshes may be formed by braiding or weaving, and the mesh density may be controlled with tension, needle space, fiber diameter, and the like. The fibers may also be coated (co-extruded, spray coated or dip (immersion, gap, curtain or otherwise) coated), as discussed in detail in several references incorporated herein.

In another variation, when the biodegradable polymer is poly(lactide-co-glycolide), poly(lactide), or poly(glycolide), the polymer has an intrinsic viscosity of from about 0.15 to about 1.5 dL/g, from about 0.25 to about 1.5 dL/g, from about 0.25 to about 1.0 dL/g, from about 0.25 to about 0.8 dL/g, from about 0.25 to about 0.6 dL/g, or from about 0.25 to about 0.4 dL/g as measured in chloroform at a concentration of 0.5 g/dL at 30°C. Various solvents, plasticizers, porosigens and other excipients may be added to the polymer in order to impact biodegradation and drug release rate. For factors affecting the degradation rate, see, e.g., Tracy et al. Factors Affecting the Degradation Rate of Poly(lactide-co-glycolide) Microspheres in vivo and in vitro. Biomaterials 20:1057-1062 (1999) and Wu XS and Wang N, Synthesis, Characterization, Biodegradation, and Drug Delivery Application of Biodegradable lactic/glycolic acid Polymers. Part II: Biodegradation. J. Biomater. Sci. Polymer Edn. 12(1):21-34(2001).

If a nonbiodegradable polymer is used to make or incorporate into the device or composition, suitable nonbiodegradable polymers include, but are not limited to, poly(ethylene vinyl acetate), poly(vinyl acetate), silicone polymers, polyurethanes, polysaccharides such as a cellulosic polymers and cellulose derivatives, acyl substituted cellulose acetates and derivatives thereof, copolymers of poly(ethylene glycol) and poly(butylene terephthalate), polystyrenes, polyvinyl chloride, polyvinyl fluoride, poly(vinyl imidazole), chorosulphonated polyolefins, polyethylene oxide, and copolymers and blends thereof.

Furthermore, the devices, or any portion thereof, may be made from any biocompatible, biodegradable or nonbiodegradable polymer that is mucoadhesive. In some instances, the device may be coated with a mucoadhesive, which may or may not be a polymer (for example, solid and semi-solid materials, such as hydrogels, e.g., comprised for example of PLG polymers, polyacrylic acids (Noveon™, Carbopol™), carageenan, alginate, xantham gum, carboxymethylcellulose, hydroxypropyl cellulose, chitins, chitosan, hyaluronic acids, lectins and their derivatives). Several theories of the microscopic nature of mucoadhesion exist; the adsorption, diffusion, electronic, fracture and wetting theories all explain some of the desirable characteristics of a mucoadhesive material. Empirically, the strength of the mucoadhesiveness is related to biomaterial characteristics (type of formulation, hydration and swelling characteristics, molecular mass, concentration, and chemical structure including functional groups, charge, ionization, chain flexibility, crosslinking density and spatial orientation), environmental characteristics (hydration condition, swelling, environmental pH, contact time and applied pressure), and physiological characteristics (mucociliary clearance, mucus turnover, and disease states) as described in Ugwoke MI et al. The Biopharmaceutical Aspects of Nasal Mucoadhesive Drug Delivery. Journal of Pharmacy and Pharmacology 53 : 3-22 (2001); Edsman K and Hägerström H. Pharmaceutical Applications of Mucoadhesion for the Non-Oral Routes. Journal of Pharmacy and Pharmacology 57: 3-22 (2005); Woodley J. Bioadhesion: New Possibilities for Drug Administration? Clin Pharmacokinet 40(2):77-84 (2001); Harikarnpakdee et al. Spraydried Mucoadhesive Microspheres: Preparations and Transport Through Nasal Cell Monolayer. AAPS PharmSciTech 7(1): E1-10 (2006); Chowdary KPR and Rao YS Mucoadhesive Microspheres for Controlled Drug Delivery. Biol. Pharm. Bull. 27(11):1717-1724 (2004); Gavini et al. Mucoashesive Microspheres for Nasal Administration of an Antiemetic Drug, Metoclopramide: in vitro/ex-vivo Studies. Journal of Pharmacy and Pharmacology 57: 287-294 (2005); Jain et al. Development and Characterization of Mucoadhesive Microspheres Bearing Salbutamol for Nasal Delivery. Drug Delivery 11:113-122 (2004); Peppas NA and Huang Y. Nanoscale Technology of Mucoadhesive Interactions. Advanced Drug Delivery Reviews 56:1675-1687 (2004); and Jasti et al. Recent Advances in Mucoadhesive Drug Delivery Systems. Business Briefing: Pharmatech (2003). The biomaterial chosen can often be optimized for the above characteristics, further enhancing mucoadhesion. For example, the mucoadhesive may absorb water to swell and become adhesive. The mucoadhesiveness and expansion of such a device can be used to facilitate fixation within the OMC. The devices may also be made from a polymer that carries a charge.

Mucoadhesive properties may also be imparted by the macroscopic mechanical construction of the devices, particularly the surface area to total mass (not average molecular weights) ratio for the device (or mucoadhesive portion therof), and specifically the interfacial surface area (the area of the device that directly contacts the mucosa or is otherwise in contact with the fluid phase of the mucus) to total supported (by mucoadhesion) mass ratios. For a particular material, within a given set of biomaterial, environmental, and physiological characteristics, different cross-sectional profiles that have higher surface area to lesser mass are often most useful to optimize mucoadhesion. These ratios, and the mucoadhesive effects, change as biodegradable materials degrade and their biomaterial characteristics change, which can also be exploited in balancing the residence time and clearance speeds.

In another variation, natural polymers may be used. Representative natural polymers that may be included in the devices include, but are not limited to, proteins, such as zein, modified zein, lectins, casein, gelatin, gluten, serum albumin, collagen and their derivatives, and polysaccharides, such as cellulose, chitin, chitosan, dextrans, polyhyaluronic acid and their derivatives. Hydrogel or sol-gel mixtures of polysaccharides may also be employed

In some variations, the devices (or any portion thereof) may be made from a metal. Examples of suitable metals include, but are not limited to, cobalt, chromium, nickel, platinum, stainless steel, titanium, tantalum, and any of their alloys, e.g., nickel-titanium alloys, and combinations thereof. Furthermore, combinations of metal and polymeric devices, as in polymer coated nitinol structures, are also claimed.

### II. METHODS

Methods for treating sinusitis and its related respiratory conditions are also described. In general, the method involves placing a device as herein described within the OMC which delivers a therapeutically effective amount of the active agent locally to the OMC over a sustained period of time. The active agent may be delivered to any structure or tissue of the OMC depending on the device used, as described above. For example, the active agent may be delivered to the uncinate process, ethmoid bulla, middle turbinate, nasal wall, or any combination thereof. The active agent may be delivered over a period of about one week, about two weeks, about three weeks, about one month, about two months, or about three months or more. Different parts of the device may be configured to deliver the active agent over different time periods. Furthermore, different parts of the device may deliver different doses of the active agent. Similarly, a combination of shorter and longer term drugs or delivery doses may be used.

The device may be placed within the OMC by active fixation methods, or a combination of active and passive fixation methods. As previously mentioned, active fixation involves any method that places a device on or within a structure of the OMC and which visually alters the OMC anatomy. For example, methods that involve puncturing, piercing, and the like can be considered active fixation. As also previously mentioned, passive fixation involves any method that places a device within the OMC without visually altering OMC anatomy. For example, these methods may include space filling, space fitting, friction fitting, and tension fitting. A tool to aid visualization, e.g., an endoscope, may also be used during placement of the device.

### III. KITS

The devices described here may be included in kits for delivering active agents to the OMC. In addition to any one of the devices described here, the kits may include a component that delivers the device or aids device delivery. For example, catheters (including guide catheters), guidewires, introducers, sheaths, and the like may be included in the kits. The catheters and guidewires may be malleable, pre-set in shape, or steerable. A lubricious coating, e.g., a Teflon™ or hydrogel coating, may also be provided on the outer or inner surface of the delivery device if desired, *see, e.g.,* Thierry et al. Bioactive Coatings of Endovascular Stents Based on Polyelectrolyte Multilayers. Biomacromolecules 4:1564-1571 (2003). Other such devices may be delivered prior to their expansion or self-assembly by using techniques such as the Proetz procedure or vertical head position lavage or the OMC area, in which case kits may include a suitable liquid carrier for the device (e.g. saline rinse). Kits for certain colloidal devices, or with colloidal components, including many of those containing gels and foams formed soon prior to their use, may further contain a separate liquid or gas component within their respective kits. The kits may also comprise a temperature, humidity, and/or pressure controlled container.

The kits may include two or more types of devices. For example, space filling or self-assembling devices may be packaged with cannulating implants. With such a kit, the cannulating implant may be used to pierce and provide a port into a target area. For example, the space filling or self-assembling devices may then be delivered through the port into a sinus ostium or sinus cavity.

Although the foregoing invention has been described in some detail by way of illustration and example for purposes of clarity of understanding, it will be readily apparent to those of ordinary skill in the art in light of the teachings of this invention that certain changes and modifications may be made thereto without departing from the spirit and scope of the appended claims.

## Claims

1. An implantable sustained drug delivery device for treating sinusitis and related respiratory conditions, comprising:
a cannula (1100, 1200, 1700) with a pointed end for piercing and cannulating one or more structures of the osteomeatal complex and one or more fixation elements for securing the cannula within the one or more structures of the osteomeatal complex; and
wherein the cannula includes a therapeutically effective amount of an active agent and is constructed and arranged to deliver the active agent for local sustained release to the osteomeatal complex.

2. The device of claim 1 wherein the respiratory condition is inflammation of the osteomeatal complex.

3. The device of claim 1 wherein the related respiratory condition is nasal or sinus polyposis.

4. The device of claim 1 wherein the active agent comprises an anti-inflammatory agent.

5. The device of claim 4 wherein the anti-inflammatory agent comprises a steroidal anti-inflammatory agent.

6. The device of claim 5 wherein the anti-inflammatory agent comprises mometasone furoate.

7. The device of claim 1 wherein the active agent is included in a coating on the device.

8. The device of claim 7 wherein the coating comprises a biodegradable polymer.

9. The device of claim 1 further comprising a biocompatible material.

10. The device of claim 1 wherein the device is configured for placement on, within, or between an uncinate process, an ethmoid bulla, a middle turbinate, a nasal wall, or
combinations thereof.

11. The device of claim 10 wherein the device is placed between the uncinate process and the ethmoid bulla.

12. The device of claim 10 wherein the device is adapted for filling the space between at least two of the uncinate process, the ethmoid bulla, the middle turbinate, and the nasal wall.

13. The device of claim 1 wherein the device is configured to pierce and secure itself within one or more structures of the osteomeatal complex.

14. The device of claim 1 wherein the biocompatible material comprises a biodegradable polymer, a nonbiodegradable polymer, a metal, or a combination thereof.

15. The device of claim 1 wherein the biocompatible material comprises a biodegradable polymer.

## Patentansprüche

1. Implantierbare Vorrichtung zur verzögerten Wirkstoffabgabe zur Behandlung von Sinusitis und verwandten respiratorischen Zuständen, umfassend:
eine Kanüle (1100, 1200, 1700) mit einem spitzen Ende zum Durchstechen und Kanülieren einer oder mehrerer Struktur(en) des osteomeatalen Komplexes und ein oder mehrerer Fixierungselement(e) zum Befestigen der Kanüle innerhalb der einen Struktur oder der mehreren Strukturen des osteomeatalen Komplexes; und
wobei die Kanüle eine therapeutisch wirksame Menge eines aktiven Mittels umfasst und konstruiert und angeordnet wird, um das aktive Mittel zur lokalen verzögerten Freisetzung an den osteomeatalen Komplex abzugeben.

2. Vorrichtung gemäß Anspruch 1, wobei der respiratorische Zustand Entzündung des osteomeatalen Komplexes ist.

3. Vorrichtung gemäß Anspruch 1, wobei der verwandte respiratorische Zustand nasale oder Sinus-Polyposis ist.

4. Vorrichtung gemäß Anspruch 1, wobei das aktive Mittel ein entzündungshemmendes Mittel umfasst.

5. Vorrichtung gemäß Anspruch 4, wobei das entzündungshemmende Mittel ein steroidales entzündungshemmendes Mittel umfasst.

6. Vorrichtung gemäß Anspruch 5, wobei das entzündungshemmende Mittel Mometasonfuroat umfasst.

7. Vorrichtung gemäß Anspruch 1, wobei das aktive Mittel in einer Beschichtung auf der Vorrichtung enthalten ist.

8. Vorrichtung gemäß Anspruch 7, wobei die Beschichtung ein biologisch abbaubares Polymer umfasst.

9. Vorrichtung gemäß Anspruch 1, die außerdem ein biokompatibles Material umfasst.

10. Vorrichtung gemäß Anspruch 1, wobei die Vorrichtung zur Platzierung auf, in oder zwischen einem Processus uncinatus, einer Bulla ethmoidalis, einer mittleren Nasenmuschel, einer Nasenwand oder Kombinationen davon konfiguriert ist.

11. Vorrichtung gemäß Anspruch 10, wobei die Vorrichtung zwischen dem Processus uncinatus und der Bulla ethmoidalis angeordnet ist.

12. Vorrichtung gemäß Anspruch 10, wobei die Vorrichtung zum Ausfüllen des Raums zwischen wenigstens zwei von dem Processus uncinatus, der Bulla ethmoidalis, der mittleren Nasenmuschel und der Nasenwand angepasst ist.

13. Vorrichtung gemäß Anspruch 1, wobei die Vorrichtung konfiguriert ist, um eine oder mehrere Struktur(en) des osteomeatalen Komplexes zu durchstechen und sich selbst darin zu befestigen.

14. Vorrichtung gemäß Anspruch 1, wobei das biokompatible Material ein biologisch abbaubares Polymer, ein biologisch nicht-abbaubares Polymer, ein Metall oder eine Kombination davon umfasst.

15. Vorrichtung gemäß Anspruch 1, wobei das biokompatible Material ein biologisch abbaubares Polymer umfasst.

## Revendications

1. Dispositif implantable d'administration prolongée de médicament pour le traitement de la sinusite et de troubles respiratoires apparentés, comprenant :
une canule (100, 1200, 1700) ayant une extrémité pointue pour percer et s'introduire dans une ou plusieurs structures du complexe ostioméatal et un ou plusieurs éléments de fixation pour fixer la canule à l'intérieur de la une ou plusieurs structures du complexe ostioméatal ; et
dans lequel la canule comprend une quantité thérapeutiquement efficace d'un agent actif et est conçue et disposée pour administrer l'agent actif pendant une libération locale prolongée au complexe ostioméatal.

2. Dispositif selon la revendication 1, dans lequel le trouble respiratoire est une inflammation du complexe ostioméatal.

3. Dispositif selon la revendication 1, dans lequel le trouble respiratoire apparenté est une polypose nasale ou sinusienne.

4. Dispositif selon la revendication 1, dans lequel l'agent actif comprend un anti-inflammatoire.

5. Dispositif selon la revendication 4, dans lequel l'anti-inflammatoire comprend un anti-inflammatoire stéroïdien.

6. Dispositif selon la revendication 5, dans lequel l'anti-inflammatoire comprend du fuorate de mométasone.

7. Dispositif selon la revendication 1, dans lequel l'agent actif est inclus dans un revêtement prévu sur le dispositif.

8. Dispositif selon la revendication 7, dans lequel le revêtement comprend un polymère biodégradable.

9. Dispositif selon la revendication 1, comprenant en outre un matériau biocompatible.

10. Dispositif selon la revendication 1, dans lequel le dispositif est conçu pour être mis en place sur, à l'intérieur, ou entre l'apophyse unciforme, la bulle ethmoïdale, le cornet moyen, la paroi nasale, ou des combinaisons de ceux-ci.

11. Dispositif selon la revendication 10, dans lequel le dispositif est placé entre l'apophyse unciforme et la bulle ethmoïdale.

12. Dispositif selon la revendication 10, dans lequel le dispositif est adapté pour remplir l'espace entre au moins deux des éléments parmi l'apophyse unciforme, la bulle ethmoïdale, le cornet moyen, et la paroi nasale.

13. Dispositif selon la revendication 1, dans lequel le dispositif est conçu pour percer et se fixer lui-même à l'intérieur d'une ou de plusieurs structures du complexe ostioméatal.

14. Dispositif selon la revendication 1, dans lequel le matériau biocompatible comprend un polymère biodégradable, un polymère non biodégradable, un métal, ou une combinaison de ceux-ci.

15. Dispositif selon la revendication 1, dans lequel le matériau biocompatible comprend un polymère biodégradable.
